(19) **Europäisches Patentamt**
**European Patent Office**
**Office européen des brevets**

(11) **EP 3 438 989 A1**

(12) # EUROPEAN PATENT APPLICATION

(43) Date of publication:
**06.02.2019 Bulletin 2019/06**

(51) Int Cl.:
***G16H 50/50*** *(2018.01)*

(21) Application number: **18154796.9**

(22) Date of filing: **01.02.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **04.08.2017 EP 17185034**

(71) Applicant: **Universität Zürich**
**8006 Zürich (CH)**

(72) Inventors:
• **Kurtcuoglu, Vartan**
**8409 Winterthur (CH)**
• **Buoso, Stefano**
**37030 Ronca' (VR) (IT)**
• **Quarteroni, Alfio**
**1025 St-Sulpice (CH)**
• **Manzoni, Andrea**
**20026 Milanese (IT)**

(74) Representative: **Veni**
**Swiss & European Patent Attorneys**
**Villa de Meuron**
**Buristrasse 21**
**3006 Bern (CH)**

(54) **METHOD AND APPARATUS FOR PREDICTING FLUID FLOW THROUGH A SUBJECT CONDUIT**

(57) A model-order reduction method is described for providing a fast fluid flow simulation model of fluid flow in a conduit such as a blood vessel with stenosis. A first method is described for generating a reduced generic numerical model (20) for predicting fluid flow characteristics of fluid flowing through a subject conduit (7'). In steps 11, 12 and 13, geometric and fluid-flow parameter data are derived from CT image scans for each sampled conduit 7 in a reference set. A 3D model is generated 13 for each sampled conduit 7. The geometric parameter data, the 3D model and the fluid flow parameter data are used to generate solutions to fluid dynamics (such as the Navier-Stokes) equations for each sampled conduit 7, and a full order model is created comprising the geometric parameters data, the fluid flow parameter data and the Navier Stokes solutions. A projection-reduction based, for example, on the Proper Orthogonal Decomposition - Discrete Empirical Interpolation Method (POD-DEIM) coupled with an offline/online splitting is used for the reduced-order description of geometric parameters, fluid parameters and fluid dynamic equations. The offline phase defines the constructors of the reduced-order model which are assembled based on the weights of the coefficients (reduced order parameters) identified in the offline phase. A second method is 30 described for using the reduced order model 20 to obtain solutions to Navier Stokes equations for the blood vessel 7' of a new patient (online phase).

Fig. 3

**Description**

Field of the invention

The invention relates to modelling of fluid flow through a subject conduit geometry such as blood flow through a blood vessel. In particular, but not exclusively, the invention relates to a method of generating a generally-applicable model for computing a pressure characteristic such as the fractional flow reserve (FFR) across a region of stenosis of a real blood vessel of a real patient.

Background of the invention

Stenosis of the arteries may require clinical or medical intervention, depending on whether the stenosis is likely to cause ischemia in the patient. Clinical intervention such as revascularization has significant attendant risks for the patient, so it is important to limit such treatment to those instances of stenosis which are likely to cause ischemia. Computer-tomographic (CT) image data of the stenotic vessel can reveal the physical characteristics of the stenosis, but does not provide a reliable basis for predicting the likelihood of ischemia. Analysis of the haemodynamic flow characteristics through the stenotic region is required in order to assess the need for clinical intervention. It has been found that the pressure gradient, or more specifically the Fractional Flow Reserve (FFR), across the obstructed region, provides a superior indicator of ischemia-causing properties of the stenosis. In simple terms, FFR represents the blood flow reduction in the branch due to the presence of the stenosis. FFR can be measured by catheterization to insert pressure transducers into the vessel, but it is expensive and carries risk of complications due to its invasive nature. High-resolution CT image data, together with physiological boundary conditions of the blood flow, have been used to construct a detailed patient-specific 3D computational model for simulating patient-specific hemodynamics and thereby calculating the FFR at simulated conditions of maximum hyperemia by computational analysis. Using the detailed anatomical model of the patient's stenosis, computation of flow and pressure can be achieved by applying mass and momentum conservation laws, for example as may be expressed by the Navier-Stokes equations. However, conventional high-fidelity solutions in three dimensions and time, such as those obtained from numerical models based on, for example, finite volume and finite element analysis, require a great deal of computing power. In published patent applications US2012041318A1 and US2013246034A1, methods are proposed for determining FFR values by computing a patient-specific model. US2014073967A1 describes a method which uses machine learning to predict, based on a database of previously-simulated synthetic cases, the FFR for a new patient under analysis. The method comprises a training phase, and a production phase in which the predictions learned in the training phase are applied to a new case. This method has advantage of being able to generate faster solutions in the production phase, but it does not permit the interrogation of the resulting solution - the predictions are presented with a statistical confidence range without the possibility of assessing errors a priori or a posteriori, for example.
It has been proposed in a paper entitled "Fast simulations of patient-specific haemodynamics of coronary artery bypass grafts based on a POD-Galerkin method and a vascular shape parameterization" by Francesco Ballarin et al, Journal of Computational Physics Vol. 315 (2016), pages 609-628, to generate a patient-specific low-order model by idealizing the patient-specific anatomy of the vessel by centerline descriptions and bifurcation angles, and by parameterizing fluid dynamic equations and selected geometry features, using this idealized anatomical model. The result is a faster, reduced-order model, which allows a "many-query" investigation, in the pre-selected parametric range, for the specific selected patient with less numerical processing. The proposed method still has the disadvantage that it requires a great deal of processing power to generate the simplified patient specific model - much more processing than can be carried out in a clinical setting. The patient-specific reduced-order model must therefore be generated "offline", in a specialist facility, using CT image data and boundary condition information from the particular blood vessel of the particular patient. Furthermore, even though the reduced-order description is patient-specific, it nevertheless relies on idealised features of the anatomical model. Idealisations can be selected so as to maximise the similarity to the patient's actual vessel geometry, but the idealisation process inevitably reduces the accuracy of the model. Even small differences can have a significant effect on the solutions of the fluid dynamics equations, and thereby on the assessment of whether or not the stenosis is likely to be ischemia-causing. A method is described in WO2016182508A1 in which stenotic blood vessels are modelled by reference to healthy vessels. The method allows faster front-end (clinical) processing but relies on an empirical, abstracted model of the stenosis and therefore discards at least some of the patient-specific information.
There is therefore a need for a method which can provide fast simulation of the fluid dynamics in a blood vessel, which can reduce the total amount of data processing, yet which remains faithful to the real patient-specific details (geometry and fluid flow boundary conditions, for example) of the blood vessel.

Brief description of the invention

It is an aim of the present invention to overcome at least some of the above disadvantages of prior art methods. To this end, the invention foresees a method according to claim 1 (referred to below as the offline method or the model generation or development method), a complementary method according to claim 12 (referred to variously as the online method, the production method or the clinical method), and a computer program product according to claim 11 and claim 15. Further variants of the invention are set out in the dependent claims.

By computing, offline, multiple high-fidelity simulations for multiple similar blood vessels, and by eg linear combination of the high-dimensional, offline-simulated solutions, a high-dimensional space is built up comprising the high-fidelity solutions. The high-dimensional space is is mapped into a lower-dimensionality subspace and paramaterized so as to build a reduced-order model which can be used as a generic numerical model for solving, within the subspace, the fluid dynamic equations for a specific patient's blood vessel at low computational cost. This enables a high-quality simulation of the specific patient's blood vessel to be carried out online, eg in a clinical setting.

As will be described below in more detail, the offline and online parts of the method are mutually complementary and are together referred to as the model-order reduction method. The offline method (generation of the high-fidelity solutions) may be carried out starting from the fluid dynamic equations discretized by means of, but not limited to, finite volumes and finite elements, such as finite volume discretisation of the unsteady incompressible Navier-Stokes equations. The resulting description is projected into a low-dimensional subspace whose basis are obtained using mathematical methods such as Proper Orthogonal Decomposition, for example. The online method (using the fast generic model to simulate fluid flow in a subject conduit such as a patient-specific stenotic blood vessel) may use hyper-reduction techniques, such as the Discrete Empirical Interpolation Method (DEIM), to efficiently retrieve the appropriate operators of the governing equations and boundary conditions as a function of physical and geometrical parameters which were determined in the parameterisation step of the offline method.

As mentioned above, the method can be used for the model-order reduction of the unsteady incompressible Navier-Stokes equations for the solution of partial differential equations using, for example, the Finite Volume method. The methodology has been applied to the model-order reduction of a model of a conduit with parametrized geometry and boundary conditions such as flow rate at inflow to the conduit. Numerical results shows that a very low number of states in the reduced-order model are required to correctly simulate the flow and they allow for a very small computational cost of the online part of the method.

The inventive methods are described here using the illustrative example of a blood vessel of a patient. It should be noted, however, that the method has application in other fields, and in particular in fields where it is impracticable to measure flow and determine derivative metrics through a particular type of conduit directly.

Detailed description of the invention

The invention will now be described in more detail with reference to the attached drawings, in which:

Figure 1 shows a prior art method of generating a reduced-order model for determining a fluid flow characteristic.

Figure 2 shows a schematic illustration of a first example of a model-order reduction method comprising a model-generation method according to the invention and an associated prediction method according to the invention.

Figure 3 shows a schematic illustration of the model-generation method of figure 2 in greater detail.

Figure 4 shows a schematic illustration of the prediction method of figure 2 in greater detail.

The drawings are provided for illustrative purposes only, as an aid to understanding certain principles underlying the invention. They should not be taken as limiting the scope of protection sought. Where the same reference numerals have been used in different figures, these are intended to refer to the same or corresponding features. However, the use of different reference numerals does not necessarily indicate the existence of a difference between the features to which the numerals refer.

In order to illustrate the inventive methods, the example will be described below of an implementation in which the inventive method provides a framework for the parametric model-order reduction of finite volume discretization of the unsteady incompressible Navier-Stokes equations. The model-order reduction method, based on the projection on a lower-order subspace obtained using Proper Orthogonal Decomposition, is based on an offline/online decomposition. The Discrete Empirical Interpolation Method is used in the online part to retrieve the non-affine/nonlinear operators and boundary conditions as function of the physical and geometrical parametrizations selected. The methodology is applied to the model-order reduction of a model of a conduit (blood vessel) with parametrized geometry and mass flow. Numerical results show that only a small number of states in the reduced-order model are required to correctly simulate the pulsatile flow, and that they permit a significant reduction in computational cost for the online (prediction) method.

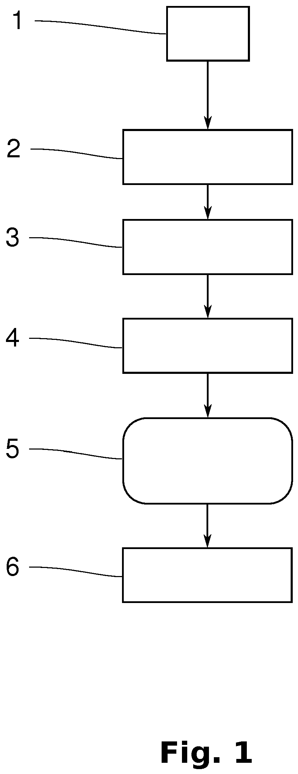

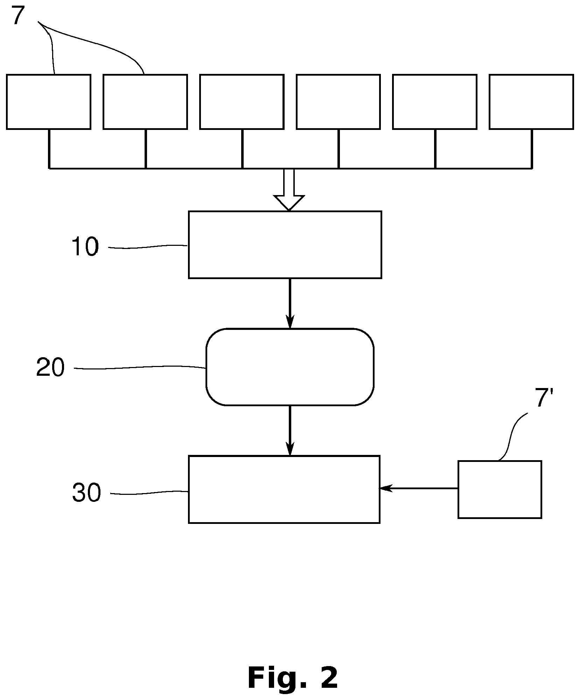

Figure 1 shows the method referred to in the article by Ballarin et al, cited above. The method comprises a first step, 1, in which CT image data of a specific patient's blood vessel under analysis are acquired. Step 2 is a segmentation of the image data at the region of interest (eg a blood vessel with stenosis, bifurcation). In step 3, the anatomical features are abstracted (idealized) to simplify the geometric description of the vessel. In step 4, the anatomy is parameterized, for example using centerlines and bifurcation angles, to describe the geometry using a limited number of parameters. The result, 5, is a reduced order model (ROM) such as a Proper Orthogonal Decomposition (eg POD-Galerkin) model which can be used to solve, in step 6, fluid dynamic equations for the blood vessel. If the boundary conditions are known for the subject conduit (blood vessel region of interest), the model could comprise only the region of interest, but in practice the boundary conditions are measured or derived at more distant locations in the flow system (such as the physical and/or pumping characteristics of the patient's heart, for example), so the model may typically include enough detail of the flow system upstream of the region of interest in order to derive the flow conditions at the inflow of the region of interest. The prior art method of figure 1 can be used to generate a reduced-order (ie fast) patient-specific model 5 for solving the Navier-Stokes equations for the particular blood vessel of the particular patient. Figure 2 shows a top-level schematic of the example methods according to the invention. In the first (offline) method 10, CT image data 7 (for example) is acquired for each of a plurality of similar blood vessels, of a plurality of patients, which will act as the reference set (also referred to as the set of snapshots). The CT image data is segmented and parameterized, for example by using Proper Orthogonal Decomposition or a Discrete Interpolation Method, and the parameterized models of each blood vessel are mapped, using a common set of reduced-order parameters, into a Reduced Order Model 20. The reduced order model (ROM) 20 represents a generic model which can be used to solve fluid dynamic equations for any blood vessel having sufficient similarity to the set of reference blood vessels 7, and which is susceptible of being described using the reduced-order parameter set of the ROM. The generic model may be provided in the form of precompiled software. The precompiled ROM can then be run on a standard desktop computer in a clinical environment to simulate fluid flow conditions in a new blood vessel of a new patient 7', for example by solving the unsteady incompressible Navier-Stokes equations, and thereby to assess an FFR value, for example. Geometric data from the scan of each new patient can then be added to the reference set, thereby improving the model.

The ROM 20 generated by the offline method 30 comprises multiple values of the reduced plurality of parameters in the common parameter space, with corresponding solutions to fluid flow solutions resulting from the simulations carried out for each sample blood vessel. The solutions can for example be expressed as fluid state variables and operators of fluid dynamic equations such as those from the unsteady incompressible Navier-Stokes equations. The term fluid dynamic equation is used here to refer generally to mass conservation, energy conservation or momentum conservation equations, for example. The example implementation described here uses CT image data, but other kinds of imaging such as MRI could be used.

As described above, the model 20 can be improved by adding each new subject conduit to the reference set and re-compiling or incrementally compiling the model with the new conduit's geometric and fluid flow data. Alternatively, or in addition, the model can be improved by calculating additional population points in the parametric space.

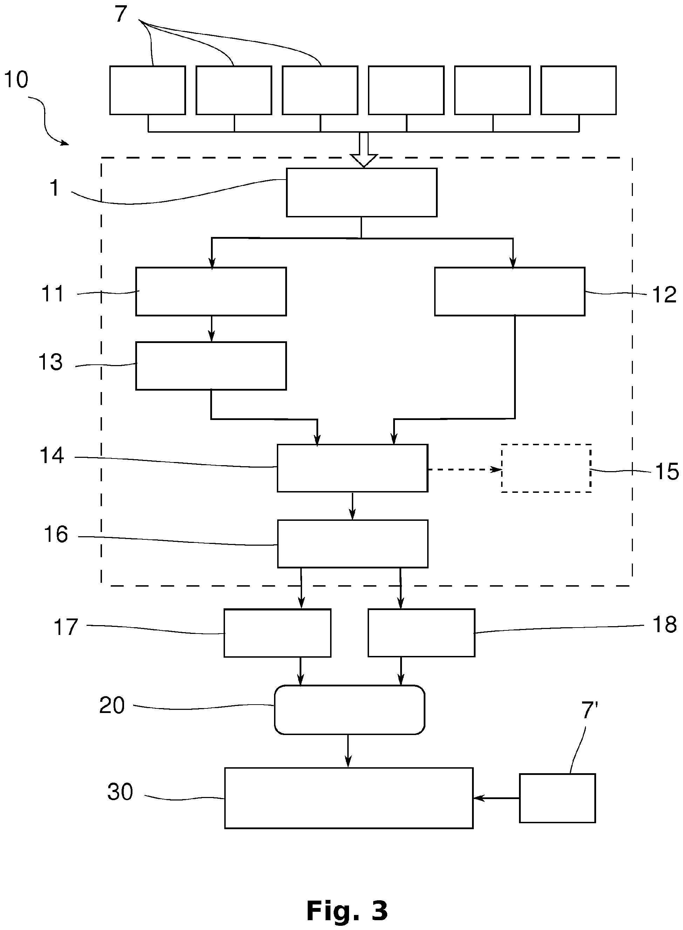

Figure 3 shows the offline method 10 in more detail. The steps within the dashed line are performed for each sampled conduit (blood vessel) 7 in order to create the numerical ROM 20. In step 1, the CT image data and measured clinical data of the blood vessel are acquired. In step 11, the image data undergoes segmentation and the vessel(s) of interest is/are identified. Predetermined geometric parameters which are relevant to the particular blood vessel and/or patient condition are then characterized. The geometric parameters may include, for example, the topology, bifurcations, linear dimensions, conduit curvature, cross-sectional area, taper etc of the particular region of interest of the vessel(s). In step 13, a 3D model is built for the segmented vessel identified. The 3D model may be a mesh or other representation. Fluid flow data is captured in step 12 and may include such measured parameters as diastolic and/or systolic blood pressure, heart stroke volume of the patient, hematocrit, viscosity, flowrate, temperature etc - parameters which can be used to define blood flow and pressure in the vessel.

In step 14, a fluid dynamic (eg hemodynamic) simulation is carried out based on the 3D geometrical model and the fluid flow parameters captured in step 12. In addition, further fluid flow parameters may be derived from the geometric model and the measured fluid flow parameters, and these may also be used in the simulations. Boundary conditions and derived fluid flow parameters may be included in the simulation, for example, such as flow rate or pressure distribution. The simulation results may be represented as the solutions to fluid dynamics equations such as the Navier-Stokes equations. At this stage, the simulation results for the particular blood vessel 7 may optionally be assessed in step 15 for the severity of stenosis by calculating an FFR value, for example. If a surgical procedure had also been carried out in order to measure an FFR value for the particular patient/blood vessel, then the simulated FFR value from step 15 may be compared with the measured value and the geometric and/or fluid flow parameters adjusted to account for any discrepancy.

In step 16, the geometric parameters, the 3D model, the measured fluid flow parameters and the fluid dynamic equation solutions for the particular vessel are added to a database. In this way, when the steps 11 to 14 and 16 have been carried out for each conduit 7 in the reference set, the database contains a full order model (FOM) of the measured, derived and simulated parameters in a common parameter space. Steps 17 and 18 concern the model order reduction which will define the description of geometry and flow of a patient specific conduit in a low-dimensional subspace. The choice of the reduced parameter and their basis can for example be carried out using dimensionality reduction methods, such as Proper Orthogonal Decomposition (POD) or Greedy algorithms. In step 17, geometric reduced order parameters are determined from the geometric parameters of the full order model (segmentation results) and the 3D model parameters. A mapping method between the full and reduced order geometric data is identified and stored for the parameter space of the reduced order model 20. In step 18, the fluid reduced order parameters are determined from the fluid flow parameters of the full order model and the solutions of the fluid dynamics equations. A mapping method between the full and reduced order fluid flow parameters is defined and stored for the parameter space of the reduced order model 20. The resulting ROM 20 can be compiled and made available to a clinical setting for simulating fluid flow in other blood vessels which have geometric and fluid-flow parameters in the variability range of the reference set.

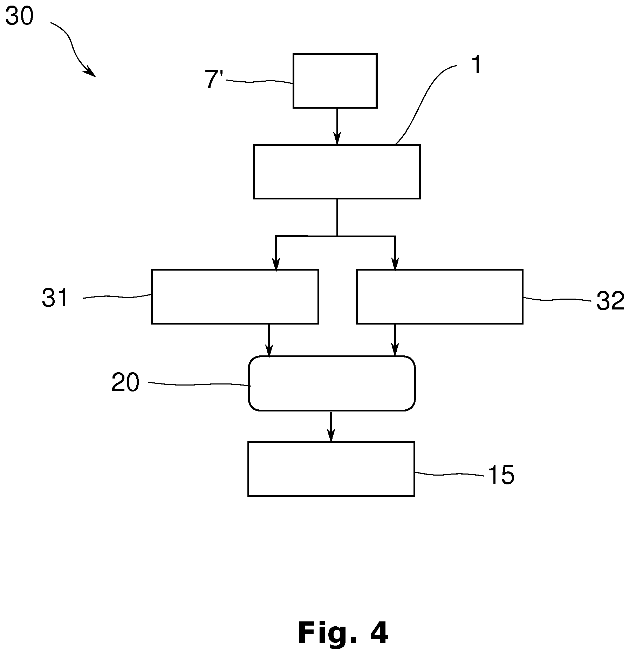

Figure 4 shows more detail of the online method 30. Steps 1, 31 and 32 are analogous to the steps 1, 11 and 12 of figure 3. In step 1, CT image data and other measure clinical data are captured for the blood vessel of a new patient. In step 31, the image data undergoes segmentation and the region(s) of the vessel(s) of interest is/are identified. Predetermined geometric parameters which are relevant to the particular blood vessel and/or patient condition are then characterized. The geometric parameters may include, for example, the topology, bifurcations, linear dimensions, conduit curvature, cross-sectional area, taper etc of the particular region of interest of the vessel(s).

Advantageously, the geometric and fluid flow parameters used for the online method are those determined in the model-order reduction step of the offline method. Similarly, the parameterization algorithm (eg POD-DEIM) used in the offline method is advantageously that used in the offline method.

Fluid flow data is captured in step 32 and may include such measured parameters as diastolic and/or systolic blood pressure, heart stroke volume of the patient, hematocrit, viscosity, flow rate, temperature etc - parameters which can be used to define blood flow and pressure. The geometric and fluid flow parameters for the new blood vessel are input into the Reduced Order Model. Using the mapping functions of steps 17 and 18 and the geometric and fluid reduced order parameters in step 15, the fluid dynamic operators, boundary conditions and equation solutions for the blood flow in the vessel are derived, which can be used to calculate an FFR value across a stenosis, for example. The geometric and fluid flow data for the new patient can also be added to the reference set, for example by re-performing the steps 1-16 and 17, 18 of figure 3 and recompiling the ROM 20.

Below is a mathematical description of the example implementation of the methods of the invention.

For an incompressible Newtonian fluid, with uniform viscosity, the momentum equations in a domain $\Omega$ with boundary $\partial\Omega$ are:

$$
\begin{aligned}
\boldsymbol{u}_t + (\boldsymbol{u}\cdot\nabla)\boldsymbol{u} - v\Delta\boldsymbol{u} &= -\nabla p && \text{in} && \Omega \\
\nabla\cdot\boldsymbol{u} &= 0 && \text{in} && \Omega \\
\boldsymbol{u} &= \boldsymbol{g} && \text{in} && \partial\Omega_D \\
v(\nabla\boldsymbol{u})\boldsymbol{n} - p\,\boldsymbol{n} &= \boldsymbol{f} && \text{in} && \partial\Omega_N
\end{aligned}
\tag{1}
$$

Here, $\boldsymbol{u}$, $p$ and $v$ are the fluid velocity vector, pressure and viscosity respectively, $\boldsymbol{u}_t$ refers to the derivative of the velocity respect time and $n$ to the normal direction of the boundary. In Eq. 1, $\boldsymbol{g}$ and $\boldsymbol{f}$ represent the Dirichlet and Neumann conditions on boundaries $\Omega_D$ and $\partial\Omega_N$, respectively.

When considering the Finite Volumes (FV) discretization of Equation 1, above, the fluid domain is subdivided into individual elements, or cells, where the equations are integrated in the corresponding volume, yielding a discrete system with dimensionality H.

As described above, the Reduced Basis (model order reduction) approach for the parametrized Navier-Stokes equations is based on the splitting of the computational effort in two steps: in the first one, the offline phase, the basis of the projection space are calculated and the Discrete Empirical Interpolation Method (DEIM) is used for the parametric description of the discrete operators of the fluid dynamic equations of the full order model (FOM). This phase involves the majority of the computational effort of the method, but is done only once. The online phase, instead, is performed every time the ROM is used for a new simulation, but it requires a significantly smaller computational effort as compared to the FOM. In this phase the dependency on the selected parameters is introduced in the description. The reduction of the computational cost of the online phase allows the speed up of the calculations in practical applications.

In the example implementation, the projection subspace is composed by Proper Orthogonal Decomposition (POD) modes computed from the solutions of the FOM for randomly selected combinations of the physical and geometrical parameters characterizing the problem. Given a time-dependent variable field $y(\boldsymbol{x}, t)$ defined at cell centroids with coor-

dinates $\boldsymbol{x}$, and being $\varphi_i(\boldsymbol{x})$ and $\alpha_i(t)$ its POD modes with relative amplitudes, it is possible to approximate the variable field with its POD reconstruction $y_r(\boldsymbol{x},t)$ as

$$y\left(\boldsymbol{x},t\right)\approx y_r\left(\boldsymbol{x},t\right)=\sum_{i=1}^{N}\varphi_i\left(\boldsymbol{x}\right)a_i\left(t\right)$$

where N is the number of POD basis selected. Given an operator defined in the FOM, $A_H$, its projection onto the selected reduced space is

$$A_N=\Phi^T X_H A_H \Phi$$

where $\Phi$ is the projection matrix onto the POD subspace whose columns are the POD mode, $\Phi=[\varphi_1,\varphi_2...\ \varphi_N]$ and $X_H$ is a suitable norm. In this case, being N << H, the dimension of the projected operator is much smaller and the reduction can lead to a computational advantage during the solution process.

For the reduction of the unsteady incompressible Navier-Stokes equations in finite volume, the POD modes of the velocity and pressure vectors denoted with $\varphi_i(\boldsymbol{x})$ and $\gamma_i(\boldsymbol{x})$ can be computed respectively. These may be calculated with the snapshot method.

A further gain in efficiency can be obtained using the Discrete Empirical Interpolation Method (DEIM) to retrieve the matrices of the discrete operators in the reduced-space without the burden of their computation in the FOM. With DEIM, each operator is expressed as a combination of parameter independent terms weighted by appositely computed coefficients. DEIM can account for the nonlinear dependency to the parameters selected. Considering a generic operator $A_H(\boldsymbol{\mu})$ function of the vector of parameters $\mu=[\mu_1,...\ ,\mu_K]$, using DEIM it can be expressed as

$$C_H\left(\mu\right)\approx Qd\left(\mu\right)$$

where $d(\mu)$ are parameter dependent coefficients of the low dimensional space defined by Q=$[\rho_1,...,\rho_M]$ whose columns, $\rho_i$, are $\mu$-independent basis. These are obtained by applying POD to a set of snapshots of the operators for sampled combinations of the entries of $\mu$ and for which the relative coefficients are then determined. During the offline phase, the basis of the subspace are calculated and, in the online part, the components of the $d(\mu)$ vector are calculated from the values of the geometric and fluid reduced order parameters of the new case. The calculation of these values usually involves the definition of a subset of cells from the full-order discretized model commonly referred to as Reduced Mesh.

The efficient generation of the Reduce Mesh is another important part to speed up the online phase of the method. In this work, the reconstruction of the Reduced Mesh of the online phase is done through the application of POD-DEIM to the mesh descriptions of the high-fidelity cases used during the training of the model. This is the outcome of the parametrization and dimensionality reduction step. The coordinates of the sampling points on the segmented new geometry are then used as weights in the POD-DEIM method to reconstruct the mesh subset needed for the online phase of the model-order reduction method.

**Claims**

1. Computer-implemented method (10) of generating a numerical model (20) for predicting fluid flow characteristics of fluid flowing through a subject conduit (7'), the method comprising:

- a first parameterization step (11) of providing geometric parameter data for each of a plurality of sampled conduits (7), the sampled conduits forming a reference set having geometrical characteristics within a predetermined geometric variance range;
- a fluid volume model generation step (13) of generating, from the geometric parameter data, a three-dimensional model of the said each sampled conduit, the three-dimensional model comprising at least one of: surface mesh or spline data of the sampled conduit wall, topological data of the sample conduit topology;
- a second parameterization step (12) of providing measured fluid flow parameter data for each of the sampled conduits (7) of the reference set, the fluid flow parameter data including at least one of: pressure of the fluid in the conduit; fluid pressure distribution in the conduit; viscosity of the fluid in the conduit; viscosity distribution of the fluid in the conduit; pumping characteristics of a pump urging the fluid through the conduit; composition of the fluid; one or more boundary conditions of the fluid flow at an inlet to the conduit;
- a fluid dynamic simulation step (14) of, using geometric parameter data and fluid flow parameter data of each sampled conduit (7), determining solutions to fluid dynamics equations for the fluid flow through the said each sampled conduit (7);
- a first model order reduction step (17) of determining a reduced plurality of geometric parameters which characterize the conduit geometries of the reference set, and mapping the geometric parameter data of the reference set to the reduced plurality of geometric parameters;
- a second model order reduction step (18) of determining a reduced plurality of fluid flow parameters which characterize fluid flow through the conduit geometries of the reference set, and mapping the fluid flow parameter data of the reference set to the reduced plurality of fluid flow parameters;
- generating a reduced order model (20) comprising the reduced plurality of geometric and fluid flow parameters of the sampled conduits (7') of the reference set, and the solutions to fluid dynamics equations for fluid flow in the sampled conduits (7') of the reference set.

2. Method according to claim 1, wherein the first parameterizing step (11) comprises acquiring image scan data of the sampled conduit and segmenting the image data, isolating the geometry of the sampled conduit in the image scan data.

3. Method according to claim 2, wherein the first parameterizing step (11) comprises automatically identifying geometric parameters of the geometry of the sampled conduit which determine fluid flow characteristics of the sampled conduit.

4. Method according to one of claims 1 to 3, comprising a step of determining the reduced plurality of geometric parameters which best characterize the conduit geometries of the reference set, and/or a step of determining the reduced plurality of fluid flow parameters which best characterize fluid flow through the conduit geometries of the reference set, wherein one or both of the steps of determining of reduced plurality of parameters comprise a proper orthogonal decomposition operation.

5. Method according to one of claims 1 to 4, wherein the fluid dynamics equations comprise unsteady incompressible Navier-Stokes equations.

6. Method according to one of the preceding claims, wherein the conduits are blood vessels with stenosis, and wherein the geometric parameters comprise geometric parameters of the stenosis.

7. Method according to claim 6 comprising determining (15), from the solutions to the fluid dynamics equations of a particular sampled conduit, a fractional flow reserve value across the stenosis of the particular sampled conduit.

8. Method according to one of the preceding claims, comprising a step of compiling the reduced order model (20) for running on a desktop computer.

9. Method according to one of the preceding claims, wherein the fluid dynamic equations are partial differential equations, and wherein the solutions to the fluid dynamic equations comprise discrete operators of the partial differential equations.

10. Method according to claim 9, comprising a discrete empirical interpolation step for determining the discrete operators.

11. A computer program product comprising computer-executable instructions for performing the method according to one of the preceding claims.

12. Computer-implemented method (30) of predicting fluid-flow parameters of a fluid flowing through a subject conduit (7') using a reduced order model generated by a model-generation method (10) according to one of claims 1 to 11, the subject conduit (7') having geometric parameters within the said geometric variance range, the prediction method (30) comprising:

- acquiring (1) geometric and fluid flow parameter data of the subject conduit (7');
- a first transformation step (31) of mapping the geometric parameter data of the subject conduit (7') on to the reduced plurality of geometric parameters determined in the first model order reduction step;
- a third parameterization step (32) of mapping the fluid flow parameter data of the subject conduit (7') on to the reduced plurality of fluid flow parameters determined in the second model order reduction step;

- determining (15), by performing a fluid dynamic simulation of the fluid flow in the subject conduit (7') using the reduced order model with the mapped geometric and flow parameters, solutions to the said fluid dynamic equations for the fluid flow in the subject conduit (7').

13. Method according to claim 12, wherein the conduits are blood vessels with stenosis, and wherein the geometric parameters comprise geometric parameters of the stenosis.

14. Method according to claim 13 comprising a step (15) of determining, from the said solutions to the fluid dynamics equations of the subject conduit, a fractional flow reserve value across the stenosis of the subject conduit.

15. A computer program product comprising computer-executable instructions for performing a method according to one of the claims 12 to 14.

1

2

3

4

5

6

# Fig. 1

**Fig. 2**

**Fig. 3**

30

7'

1

31

32

20

15

**Fig. 4**

Europäisches Patentamt

European Patent Office

Office européen des brevets

## EUROPEAN SEARCH REPORT

Application Number

EP 18 15 4796

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BALLARIN FRANCESCO ET AL: "Fast simulations of patient-specific haemodynamics of coronary artery bypass grafts based on a POD-Galerkin method and a vascular shape parametrization", JOURNAL OF COMPUTATIONAL PHYSICS, LONDON, GB, vol. 315, 5 April 2016 (2016-04-05), pages 609-628, XP029525360, ISSN: 0021-9991, DOI: 10.1016/J.JCP.2016.03.065 * abstract * * page 610, paragraph 4 - page 625, paragraph 2 * ----- | 1-15 | INV. G16H50/50 |

TECHNICAL FIELDS SEARCHED (IPC)

G16H

The present search report has been drawn up for all claims

| Place of search | Date of completion of the search | Examiner |
|---|---|---|
| Munich | 17 August 2018 | Menschner, Philipp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012041318 A1
- US 2013246034 A1
- US 2014073967 A1
- WO 2016182508 A1

**Non-patent literature cited in the description**

- **FRANCESCO BALLARIN et al.** Fast simulations of patient-specific haemodynamics of coronary artery bypass grafts based on a POD-Galerkin method and a vascular shape parameterization. *Journal of Computational Physics,* 2016, vol. 315, 609-628